Europäisches Patentamt

European Patent Office

Office européen des brevets

(19)

(11) Veröffentlichungsnummer : **0 029 409**
**B1**

(12) # EUROPÄISCHE PATENTSCHRIFT

(45) Veröffentlichungstag der Patentschrift :
15.08.84

(51) Int. Cl.³ : **C 07 D223/22**

(21) Anmeldenummer : **80810321.2**

(22) Anmeldetag : **24.10.80**

(54) **Verfahren zur Herstellung von 5-Cyano-5H-dibenz(b,f)azepin und 5H-Dibenz(b,f)azepin-5-carboxamid.**

(30) Priorität : 30.10.79 CH 9705/79

(43) Veröffentlichungstag der Anmeldung :
27.05.81 Patentblatt 81/21

(45) Bekanntmachung des Hinweises auf die Patenterteilung : 15.08.84 Patentblatt 84/33

(84) Benannte Vertragsstaaten :
**BE CH DE FR GB IT LI NL SE**

(56) Entgegenhaltungen :
**DD-A- 133 053**
**DE-C- 1 136 707**
**HOUBEN-WEYL "Methoden der organischen Chemie" 4. Auflage, Band 8 1952, GEORG THIEME VERLAG, Stuttgart Seite 173**
**Patents Abstracts of Japan, Band 1, Nr. 70, 8. Juli 1977 Seite 1232C77**
**Chemical Abstracts, Band 79, Nr. 17 29. Oktober 1973 Columbus, Ohio, USA T. SARAIE et al. "new synthesis of nitriles" Seite 396, Spalte 2, Abstract Nr. 104882m**

(73) Patentinhaber : **CIBA-GEIGY AG**
**Postfach**
**CH-4002 Basel (CH)**

(72) Erfinder : **Aufderhaar, Ernst, Dr.**
**Spiegelgrund 4**
**CH-4303 Kaiseraugst (CH)**
Erfinder : **Sprecher, Klemenz**
**Müllheimerstrasse 176**
**CH-4057 Basel (CH)**
Erfinder : **Zergényi, Janos Dr.**
**Jurastrasse 31**
**CH-4411 Seltisberg (CH)**

## Beschreibung

Die Erfindung betrifft ein neues und technisch fortschrittliches Verfahren zur Herstellung von 5-Cyano-5H-dibenz [b,f] azepin, welches dadurch gekennzeichnet, ist, dass man 5H-Dibenz [b,f] azepin mit einem Halogencyan in Gegenwart von stark polaren Substanzen umsetzt. Halogencyan steht für Jodcyan, insbesondere aber für Chlor- oder Bromcyan.

Gemäss Tetrahedron Letters, 1973, Seite 2121-2124 kann 5-Cyano-5H-dibenz [b,f] azepin aus 5-Carboxamido-5H-dibenz [b,f] azepin durch Wasserabspaltung mittels eines Gemisches von Triäthylbenzylammoniumchlorid, Chloroform und 50 %-iger wässeriger Natronlauge erhalten werden. Der Nachteil dieses Verfahrens besteht darin, dass das benötigte Ausgangsmaterial üblicherweise erst durch Umsetzung von 5H-Dibenz [b,f] azepin mit Phosgen und anschliessender Umsetzung des erhaltenen Carbonylchlorids mit Ammoniak hergestellt werden muss. Aus Houben-Weyl, Vol. VIII, Seite 173 ist ein günstiges Verfahren zur Darstellung von substituierten Cyanamiden bekannt, welches in der Einwirkung von Halogencyan auf ein Amin besteht. Um die Bildung von Guanidinen zu verhindern, wird die Umsetzung bei tiefer Temperatur durchgeführt. Die entstehende Halogenwasserstoffsäure muss entweder durch einen berechneten Ueberschuss von Amin oder durch Zugabe von Alkali abgefangen werden. So wird z. B. die Umsetzung von N-Cyclohexyl-N-methylamin als sekundärem Amin im Ueberschuss mit Chlorcyan in Benzol bei einer Temperatur von 0-5° zum N-Cyclohexyl-N-methylcyanamid beschrieben.

Die Uebertragung solcher Reaktionsbedingungen auf die Umsetzung von 5H-Dibenz [b,f] azepin mit z. B. Chlorcyan ergab jedoch kein 5-Cyano-5H-dibenz [b,f] azepin, so dass die Lösung der gestellten Aufgabe, 5-Cyano-5H-dibenz [b,f] azepin aus 5H-Dibenz [b,f] azepin und einem Halogencyan in Analogie zu bekannten Methoden herzustellen, nicht möglich war.

Ueberraschenderweise wurde nun gefunden, dass man die erfindungsgemässe Umsetzung in Gegenwart von stark polaren Substanzen durchführen kann, wobei die Nachteile, in Gegenwart von überschüssigem Amin oder zusätzlichem Alkali und ausserdem noch bei tiefer Temperatur arbeiten zu müssen, vermieden werden.

Entsprechend ihren physikalischen-chemischen Eigenschaften können solche stark polaren Substanzen zugleich als Lösungsmittel dienen, denen man gegebenenfalls ein zusätzliches Lösungsmittel unpolaren Charakters zusetzen kann. Hierbei variieren die Mengenverhältnisse an stark polarer Substanz zu weiterem unpolaren Lösungsmittel in weiten Grenzen, indem einerseits eine oder ein Gemisch von stark polaren Substanzen gegebenenfalls als alleiniges Lösungsmittel, andererseits im Gemisch mit einem unpolaren Lösungsmittel bis zu katalytisch wirksamen Mengen eingesetzt werden kann. Die Reaktionstemperatur liegt im Bereich von 20-100°, vorzugsweise von 50-80°.

Stark polare Verbindungen sind z. B. aliphatische N-Niederalkyl- oder N,N-Diniederalkyl-amide von Carbonsäuren der Formel R—COOH (I), worin Niederalkyl jeweils durch Niederalkoxy substituiert sein kann, und Niederalkyl oder Niederalkoxy jeweils bis zu 7, insbesondere bis zu 4 Kohlenstoffatome aufweist, und R für Niederalkyl mit bis zu 5, insbesondere bis zu 3 Kohlenstoffatomen steht.

Demnach sind aliphatische N-Niederalkyl- bzw. N-Niederalkoxyniederalkylamide von Niederalkancarbonsäuren beispielsweise N-Methylacetamid, N-Methylpropionamid bzw. N-Methoxymethylacetamid oder N-Methoxymethylpropionamid, während als N,N-Diniederalkyl- bzw. N-Niederalkyl-N-niederalkoxyniederalkyl- bzw. N,N-Diniederalkoxyniederalkylamide von Niederalkancarbonsäuren beispielsweise N,N-Dimethylacetamid, N,N-Dimethylpropionamid bzw. N-Methyl-N-methoxymethylacetamid, N-Methyl-N-methoxymethylpropionamid, bzw. N,N-Di-(methoxymethyl)-acetamid oder N,N-Di-(methoxymethyl)-propionamid genannt werden. Weitere stark polare Substanzen leiten sich von N-Niederalkylphosphorsäureamiden ab, worin Niederalkyl bis zu 4 Kohlenstoffatome hat, und stellen insbesondere Hexamethyl- bzw. Hexaäthylphosphorsäuretriamid dar.

Stark polare Verbindungen sind ferner gegebenenfalls N-niederalkylierte, worin Niederalkyl bis zu 4 Kohlenstoffatome hat, wie N-methylierte, cyclische Carbonsäureimide mit z. B. 5-7 Ringgliedern, wie ein gegebenenfalls N-niederalkyliertes, wie etwa N-methyliertes 2-Oxo-alkylenimin, z. B. Pyrrolidon-(2), N-Methylpyrrolidon-(2), Piperidon-(2), N-Methyl-piperidon-(2), ε-Caprolactam oder N-Methyl-ε-Caprolactam.

Als weitere stark polare Substanzen werden u. a. polyniederalkylierter Harnstoff, wie Tetraniederalkyl-harnstoff, worin Niederalkyl bis zu 3 Kohlenstoffatome hat, z. B. Tetramethylharnstoff, Sulfolan, ferner aber auch quaternäre Ammoniumverbindungen, z. B. Mono-, Di-, Tri- oder Tetraalkylammoniumverbindungen, wobei jeder der Alkylreste bis zu 18 Kohlenstoffatome haben und durch eine Arylgruppe, wie gegebenenfalls z. B. durch Niederalkyl oder Nieder alkoxy mit jeweils bis zu 7, vorzugsweise bis zu 4 Kohlenstoffatomen, Halogen, Hydroxy oder Nitro, substituiertes Phenyl substituiert sein kann, oder deren Salze, z. B. mit Mineralsäuren, etwa Schwefelsäure oder Salzsäure, genannt.

Alkylreste der genannten Art sind demnach z. B. Methyl, Aethyl, Propyl, Isopropyl, n-Butyl, n-Pentyl, n-Hexyl, n-Decyl, Lauryl, Palmityl, Stearyl oder Oleyl. Quaternäre Ammoniumverbindungen der genannten Art und deren Salze sind demnach z. B. Stearyltrimethylammonium-, Oleyl-triäthylammonium, Lauryl-benzyldiäthylammonium-, Palmityl-benzyl-dimethylammonium-, Benzyltrimethylammonium-, Benzyltriäthylammonium, Dibenzyl-diäthylammonium-, Tribenzyl-n-propylammonium- oder Tetrabenzylammoniumhydroxid oder deren Salze, z. B. mit Mineralsäuren, etwa Halogenwasserstoffsäuren, z. B. Chlor- oder Bromwasserstoffsäure oder Schwefelsäure, oder organischen Säuren, z. B. Essigsäure. Stark polare Substanzen, etwa der genannten Art, können auch als Gemische eingesetzt werden.

Unpolare Flüssigkeiten als weitere Lösungsmittel sind z. B. halogenierte, etwa chlorierte Niederalkane z. B. Chloroform, Tetrachlorkohlenstoff, 1,2-Dichloräthan oder 1,1,1-Trichloräthan, ferner Lösungsmittel aromatischen Charakters, z. B. gegebenenfalls niederalkyliertes, wie methyliertes, oder halogeniertes, wie chloriertes Benzol, z. B. Benzol, Toluol, Xylol, oder Chlorbenzol.

Die Erfindung betrifft in erster Linie das obige Verfahren zur Herstellung von 5-Cyano-5H-dibenz [b,f] azepin, worin man als Halogencyan Chlor- oder Bromcyan und als stark polare Substanzen N,N-Diniederalkylamide von Carbonsäuren der Formel (I) verwendet, worin Niederalkyl jeweils bis zu 3 und R bis zu 2 Kohlenstoffatome hat, z. B. N,N-Dimethylacetamid, N,N-Diäthylacetamid, N-N-Di-n-propylacetamid, N,N-Dimethylpropionamid oder N,N-Diäthylpropionamid, ferner Hexamethylphosphorsäuretriamid, N-Methylpyrrolidon-(2) oder Sulfolan, oder als quaternäre Ammoniumverbindung z. B. Benzyltriäthylammoniumchlorid oder Dibenzyl-diäthylammoniumchlorid, und gegebenenfalls als unpolare weitere Lösungsmittel 1,2-Dichloräthan, 1,1,1-Trichloräthan, oder als aromatische Lösungsmittel Benzol oder Toluol verwendet.

Die Erfindung betrifft das obige Verfahren zur Herstellung von 5-Cyano-5H-dibenz [b,f] azepin, wobei man als Halogencyan Chlorcyan und als stark polare Substanzen z. B. N,N-Dimethylacetamid, N,N-Diäthylacetamid, ferner Hexamethylphosphorsäuretriamid oder Sulfolan, oder als quaternäre Ammoniumverbindung z. B. Benzyltriäthylammoniumchlorid, und gegebenenfalls als unpolare weitere Lösungsmittel 1,2-Dichloräthan, 1,1,1-Trichloräthan oder Toluol verwendet.

Die Erfindung betrifft insbesondere das in den Beispielen beschriebene Verfahren.

Die Erfindung betrifft ferner ein neues und technisch fortschrittliches Verfahren zur Herstellung von 5H-Dibenz [b,f] azepin-5-carboxamid, welches dadurch gekennzeichnet ist, dass man 5H-Dibenz [b,f] azepin mit einem Halogencyan in Gegenwart von stark polaren Substanzen zum 5-Cyano-5H-dibenz [b,f] azepin umsetzt, und dieses zum 5H-Dibenz [b,f] azepin-5-carboxamid hydrolysiert.

Die Herstellung des hierzu benötigten 5-Cyano-5H-dibenz [b,f] azepin erfolgt nach dem oben beschriebenen Verfahren. Hieran schliesst sich erfindungsgemäss die Umwandlung der 5-Cyanogruppe im 5-Cyano-5H-dibenz [b,f] azepin in die 5-Carboxamidgruppe mittels Hydrolyse an. Diese kann mittels basischer oder saurer Mittel durchgeführt werden. Als basische Mittel kommen hierzu etwa die Oxide oder Hydroxide von Erdalkali- oder Alkalimetallen, z. B. Magnesium- oder Calciumhydroxid, ferner z. B. Natriumhydroxid, gegebenenfalls in Gegenwart eines Peroxids, wie Wasserstoffperoxid, oder ein Alkalimetallbicarbonat, wie Natriumbicarbonat, im Gemisch mit Wasserstoffperoxid in Betracht, während saure Mittel z. B. Mineralsäuren, wie Schwefelsäure oder Polyphosphorsäure, ferner Niederalkan- oder Halogenniederalkancarbonsäuren mit bis zu 4 Kohlenstoffatomen, z. B. Ameisen- oder Essigsäure bzw. Trichlor- oder Trifluoressigsäure im Gemisch mit Mineralsäuren, z. B. konz. Schwefelsäure, darstellen. Saure Mittel sind ferner Lewis-Säuren, z. B. Bortrifluorid, welches als Lösung in einer Niederalkancarbonsäure der oben beschriebenen Art, wie Essigsäure, aber auch als definierte Verbindung, z. B. der Formel $BF_3 \cdot 2 \ CH_3COOH$ vorliegen kann. Gegebenenfalls fügt man dem Reaktionsgemisch noch ein weiteres Lösungsmittel, z. B. ein solches aromatischen Charakters, wie etwa Chlorbenzol zu. Die Reaktionstemperaturen liegen im Bereich von − 5 bis + 80°, vorzugsweise von 0-40°.

Aus der DE-Patentschrift 1.136.707 ist ein Verfahren zur Herstellung von 5H-Dibenz [b,f] azepin-5-carboxamid durch Umsetzung von 5H-Dibenz [b,f] azepin mit Phosgen zum 5-Chlorcarbonyl-5H-dibenz [b,f] azepin und dessen anschliessende Umsetzung mit Ammoniak zu der genannten Verbindung bekannt. Die Umsetzung des 5H-Dibenz [b,f] azepin mit Phosgen wird in Toluol zunächst bei 70°, dann unter Rückfluss durchgeführt. Die anschliessende Umsetzung dieser Verbindung mit Ammoniak erfolgt in Aethanol bei Siedetemperatur.

Demgegenüber kommt dem erfindungsgemässen Verfahren einerseits der Vorteil des oben beschriebenen technisch fortschrittlichen Verfahrens zur Herstellung des als Zwischenprodukt benötigten 5-Cyano-5H-dibenz [b,f] azepin zugute ; andererseits werden bei der Hydrolyse der 5-Cyanogruppe zur 5-Carboxamidgruppe milde Reaktionsbedingungen angewendet, die sich insbesondere auf die Reinheit des erhaltenen Endprodukts günstig auswirken. Dieses tritt besonders augenfällig bei der Durchführung der Hydrolyse mittels Bortrifluorid, z. B. in Form des definierten Komplexes mit 2 Mol Essigsäure, in Erscheinung, indem bei der bei normaler Temperatur durchgeführten Hydrolyse eine neue und bisher in der Literatur nicht beschriebene kristalline Additionsverbindung des Hydrolyseprodukts mit Bortrifluorid isoliert werden kann, welche durch nachfolgende Behandlung mit Wasser in das Endprodukt überführt wird.

Die folgenden Beispiele dienen zur Illustration der Erfindung ; Temperaturen sind in Celsiusgraden angegeben.

Beispiel 1

97,0 g 5H-Dibenz [b,f] azepin werden in 320 ml 1,2-Dichloräthan suspendiert und nach Zufügen von 18 ml Hexamethylphosphorsäuretriamid auf 70° erwärmt.

Man leitet bei dieser Temperatur 36,8 g Chlorcyan so rasch ein, dass am Kopf eines auf − 20° gekühlten Rückflusskühlers gerade kein Gasangang beobachtet wird.

Die entstandene klare hellbraune Lösung wird während weiteren 15 Std. auf 70° gehalten, dann abgekühlt und während einer Stunde mit 180 ml Wasser verrührt.

Nach der Trennung der Phasen wird die organische Lösung mit 100 ml 10 %iger Natronlauge gut durchgeschüttelt, abgetrennt und mit Wasser neutral gewaschen. Nach dem Trocknen über Natriumsulfat und Eindampfen hinterbleibt ein dunkelgrüner kristalliner Rückstand, der mit 150 ml Isopropanol aufgenommen, verrieben und abgesaugt wird. Man wäscht zweimal mit je 50 ml Isopropanol nach und erhält grünlichweisse Kristalle, die aufgrund des IR-Spektrums identisch mit authentischem 5-Cyano-5H-dibenz [b,f] azepin sind.

Fp. 103-107, 5° ; Ausbeute 100,0 g, 91,7 % d. Th.

Ein analog durchgeführter Ansatz in 1,1,1-Trichloräthan und Hexamethyl-phosphorsäuretriamid als Lösungsmittel benötigt total 48 Std. Reaktionszeit und eine auf 37,8 g erhöhte Chlorcyan-Zugabe.

Ausbeute : 105,0 g ; 96,3 % d. Th ; Fp. 105,5-107,1°.

Beispiel 2

97,0 g 5H-Dibenz [b,f] azepin werden in 320 ml 1,2-Dichloräthan suspendiert und nach Zugabe von 4 ml Hexamethyl-phosphorsäuretriamid auf 60° erwärmt.

Man setzt einen auf − 20° gekühlten Rückflusskühler auf und leitet dann im Lauf von 3 Stunden 50 g Chlorcyan ein. Anschliessend hält man unter Rühren weitere 10 Stunden auf 60°, wobei man nach 5 Stunden eine klare Lösung erhält. Man ersetzt den Rückflusskühler durch einen absteigenden Kühler und destilliert bei gleicher Temperatur durch Anlegen eines schwachen Vakuums 350 ml einer Mischung von Chlorcyan und 1,2-Dichloräthan in eine gekühlte Vorlage ab. Der abdestillierte 1,2-Dichloräthan-Anteil wird hierbei zur Entfernung von nicht umgesetztem Chlorcyan laufend durch Zufügung von frischem 1,2-Dichloräthan in den Destillationskolben ergänzt. Das Destillat wird entweder für einen weiteren Ansatz verwendet oder durch Behandeln mit wässriger Natronlauge vom Chlorcyan befreit und in üblicher Weise aufgearbeitet. Der Inhalt des Destillationskolbens wird mit 200 ml Wasser versetzt, auf Raumtemperatur abgekühlt und durch Zutropfen von 30 %iger Natronlauge bleibend alkalisch gestellt. Nach der Phasentrennung wird die organische Phase filtriert, zweimal mit Wasser gewaschen und im Vakuum eingedampft. Der Rückstand wird in Methanol aufgenommen und liefert nach einstündigem Kühlen im Eisbad 95.7 g 5-Cyano-5H-dibenz [b,f] azepin, welches aufgrund des IR-Spektrums identisch mit authentischem Material ist. Fp. 108-109°. Aus der Mutterlauge kann eine zweite Kristallisation von 2,5 g erhalten werden. Totalausbeute 98,2 g, 90 % d. Th.

Beispiel 3

97,0 g 5H-Dibenz [b,f] azepin werden in 320 ml N,N-Dimethy acetamid, gelöst, und analog der Arbeitsweise des Beispiels 1 bei 30° im Laufe von 110 Min. 40,0 g Chlorcyan eingeleitet. Man heizt im Laufe von 120 Min. auf eine Temperatur von 68° auf und hält drei Stunden bei dieser Temperatur. Nach Abkühlen werden 200 ml 10 %ige Natronlauge und 500 ml Wasser zugegeben, die überstehende wässrige Phase abdekantiert und das erhaltene halbkristalline Produkt noch zweimal mit je 500 ml Wasser nachgewaschen. Der Rückstand wird in Methylenchlorid aufgenommen, die Lösung mit Wasser neutral gewaschen und nach Eindampfen in Isopropanol aufgenommen, wonach man 5-Cyano-5H-dibenz [b,f] azepin vom Fp. 108, 3-108, 7° erhält.

Ausbeute : 76,8 g, 70 % d. Th.

Beispiel 4

Analog der Arbeitsweise des Beispiels 1 werden 97,0 g 5H-Dibenz [b,f] azepin in 320 ml 1,2-Dichloräthan suspendiert. Man setzt 2 ml N,N-Dimethylacetamid zu, heizt auf 60° auf und leitet im Lauf von 2 Stunden 50 g Chlorcyan ein, wobei der Rückflusskühler auf − 17° gekühlt wird, um Chlorcyanverluste zu vermeiden. Man hält 16 Stunden bei 60°, setzt dann einen absteigenden Kühler auf und destilliert bei Normaldruck 200 ml eines Gemisches von Chlorcyan und 1,2-Dichloräthan in eine gekühlte Vorlage ab. Während der Destillation wird das übergehende 1,2-Dichloräthan laufend durch frisches Lösungsmittel ersetzt, um restliches Chlorcyan zu entfernen. Der Kolbeninhalt wird auf Raumtemperatur abgekühlt, mit 200 ml Wasser versetzt und durch Zugabe von 30 %iger Natronlauge bleibend alkalisch eingestellt. Die weitere Aufarbeitung analog Beispiel 2 liefert 96.3 g 5-Cyano-5H-dibenz [b,f] azepin, welches aufgrund des IR-Spektrums identisch ist mit authentischem Material, Fp. 108-109°.

Aus der Mutterlauge können weitere 4 g Endprodukt erhalten werden. Totalausbeute 100,3 g ; 92 % d. Th.

Beispiel 5

In eine auf 80° erwärmte Lösung von 9,64 g 5H-Dibenz [b,f] azepin und 1 g Benzyltriäthylammoniumchlorid in 40 ml Toluol werden innerhalb 1 Stunde 6 g Chlorcyan eingeleitet. Das Reaktionsgemisch wird noch 5 Stunden bei 80° weitergerührt, dann auf Raumtemperatur gekühlt und mit 40 ml 2-n. Natriumhydroxydlösung gewaschen, die organische Phase abgetrennt und diese im Vakuum zur Trockne verdampft, wonach man 5-Cyano-5H-dibenz [b,f] azepin erhält, welches mit dem nach Beispiel 1 erhältlichen Produkt identisch ist. Fp. 107-108°. Ausbeute 6,2 g ; 56,9 % d. Th.

Beispiel 6

Man versetzt eine Suspension von 10,5 g (0,05 Mol) 5-Cyano-5H-dibenz [b,f] azepin in 100 ml Chlorbenzol in einer Portion mit der Lösung von 7,7 ml des Komplexes $BF_3 \cdot 2\ CH_3COOH$ in 19 ml Essigsäure. Unter leichtem Temperaturanstieg entsteht eine klare hellgrüne Lösung, aus der nach 5 Minuten eine kristalline Additionsverbindung des 5H-Dibenz [b,f] azepin-5-carboxamids mit $BF_3$ auskristallisiert. Man rührt eine Stunde im Eisbad nach, filtriert ab und wäscht mit Chlorbenzol und Petroläther. Das kristalline Material wird in 150 ml Wasser angeschlämmt, eine Stunde bei Raumtemperatur gerührt, abfiltriert und mit Wasser neutral gewaschen. Nach Trocknen im Vakuum bei 50° erhält man 11,3 g (95,7 % d. Th.) rohes 5H-Dibenz [b,f] azepin-5-carboxamid, das aus Aethanol/Wasser umkristallisiert und mittels DC und IR-Analyse mit authentischem Material identifiziert wird.

Beispiel 7

2,0 g 5-Cyano-5H-dibenz [b,f] azepin werden in 40 ml Methanol suspendiert und bei Raumtemperatur mit 10 ml 30 %igem $H_2O_2$ versetzt. Man rührt 30 Minuten und trägt dann 10 g Natriumhydrogencarbonat portionenweise ein. Nach Rühren über Nacht bei Raumtemperatur wird mit 50 ml Wasser verdünnt, der ausgefallene Niederschlag abfiltriert und mehrmals mit Wasser nachgewaschen. Man erhält nach dem Trocknen 3,0 g (95,2 % d. Th.) 5H-Dibenz [b,f] azepin-5-carboxamid, dass mittels DC und IR-Analyse mit authentischem Material identifiziert wird.

Beispiel 8

2,0 g 5-Cyano-5H-dibenz [b,f] azepin werden portionenweise bei Raumtemperatur in die Mischung von 16 ml Essigsäure und 4 ml konz. Schwefelsäure eingetragen. Nach einstündigem Rühren ist vollständige Lösung eingetreten. Man giesst auf 200 ml Eiswasser, rührt die entstandene weise Suspension 10 Minuten, filtriert ab und wäscht den Niederschlag mit Wasser neutral. Man erhält 2,0 g (95,2 % d. Th.) 5H-Dibenz [b,f] azepin-5-carboxamid, welches nach DC und IR-Analyse mit authentischem Material identisch ist.

**Ansprüche**

1. Verfahren zur Herstellung von 5H-Dibenz [b,f] azepin-5-carboxamid, dadurch gekennzeichnet, dass man 5H-Dibenz [b,f] azepin mit einem Halogencyan in Gegenwart von stark polaren Substanzen zum 5-Cyano-5H-dibenz [b,f] azepin umsetzt und dieses hydrolysiert.

2. Verfahren nach Anspruch 1, dadurch gekennzeichnet, dass man als Halogencyan Jodcyan, insbesondere Chlor- oder Bromcyan verwendet.

3. Verfahren nach Anspruch 1, dadurch gekennzeichnet, dass man als stark polare Verbindungen aliphatische N-Niederalkyl- oder N,N-Diniederalkyl-amide von Carbonsäuren der Formel R—COOH (I) verwendet, worin Niederalkyl jeweils durch Niederalkoxy substituiert sein kann, und Niederalkyl und Niederalkoxy jeweils bis zu 7 Kohlenstoffatome aufweist und R für Niederalkyl mit bis zu 5, insbesondere bis zu 3 Kohlenstoffatomen steht.

4. Verfahren nach Anspruch 1, dadurch gekennzeichnet, dass man als stark polare Verbindungen N-Niederalkylphosphorsäureamide, worin Niederalkyl bis zu 4 Kohlenstoffatome hat, verwendet.

5. Verfahren nach Anspruch 1, dadurch gekennzeichnet, dass man als stark polare Substanzen gegebenenfalls N-niederalkylierte cyclische Carbonsäureimide mit 5-7 Ringgliedern, worin Niederalkyl bis zu 4 Kohlenstoffatome hat, verwendet.

6. Verfahren nach Anspruch 1, dadurch gekennzeichnet, dass man als stark polare Substanzen Mono-, Di-, Tri- oder Tetraalkylammoniumverbindungen, worin jeder der Alkylreste bis zu 18 Kohlenstoffatome hat und gegebenenfalls durch einen gegebenenfalls durch Niederalkyl oder Niederalkoxy mit jeweils bis zu 7 Kohlenstoffatomen, Halogen, Hydroxy oder Nitro substituierten Arylrest substituiert sein kann, oder deren Salze, oder poly-niederalkylierten Harnstoff, worin Niederalkyl bis zu 3 Kohlenstoffatome hat, oder Sulfolan verwendet.

7. Verfahren nach einem der Ansprüche 1-6, dadurch gekennzeichnet, dass man die Umsetzung im Gemisch mit einem unpolaren Lösungsmittel durchführt.

8. Verfahren nach einem der Ansprüche 1-7, dadurch gekennzeichnet, dass man als Halogencyan Chlor- oder Bromcyan und als stark polare Substanzen, N,N-Diniederalkylamide von Carbonsäuren der Formel (1) verwendet, worin Niederalkyl jeweils bis zu 3 und R bis zu 2 Kohlenstoffatome hat, ferner Hexamethylphosphorsäuretriamid, N-Methylpyrrolidon-(2) oder Sulfolan, oder als quaternäre Ammoniumverbindung Benzyltriäthylammoniumchlorid oder Dibenzyl-diäthylammoniumchlorid, und gegebenenfalls als unpolare weitere Lösungsmittel 1,2-Dichloräthan, 1,1,1-Trichloräthan, oder als aromatische Lösungsmittel Benzol oder Toluol verwendet.

9. Verfahren nach einem der Ansprüche 1-7, dadurch gekennzeichnet, dass man als Halogencyan Chlorcyan und als stark polare Substanzen N,N-Dimethylacetamid, N,N-Diäthylacetamid, ferner Hexamethylphosphorsäuretriamid oder Sulfolan, oder als quaternäre Ammoniumverbindung Benzyltriäthylammoniumchlorid, und gegebenenfalls als unpolare weitere Lösungsmittel 1,2-Dichloräthan, 1,1,1-Trichloräthan oder Toluol verwendet.

10. Verfahren nach einem der Ansprüche 1-9, dadurch gekennzeichnet, dass man die Umsetzung mit einem Halogencyan in einem Temperaturbereich von 20-100 °C durchführt.

11. Verfahren nach Anspruch 1, dadurch gekennzeichnet, dass man mittels basischer Mittel hydrolysiert.

12. Verfahren nach Anspruch 11, dadurch gekennzeichnet, dass man mittels eines Oxids oder Hydroxids von Erdalkali- oder Alkalimetallen gegebenenfalls in Gegenwart eines Peroxids hydrolysiert.

13. Verfahren nach Anspruch 12, dadurch gekennzeichnet, dass man mittels eines Alkalibicarbonats im Gemisch mit Wasserstoffperoxid hydrolysiert.

14. Verfahren nach Anspruch 1, dadurch gekennzeichnet, dass man mittels saurer Mittel hydrolysiert.

15. Verfahren nach Anspruch 1, dadurch gekennzeichnet, dass man mittels einer gegebenenfalls als Lösung in einer Niederalkancarbonsäure vorliegenden Lewissäure hydrolysiert.

16. Verfahren nach Anspruch 15, dadurch gekennzeichnet, dass man mittels der als definierte Verbindung der Formel $BF_3 \cdot 2\ CH_3COOH$ vorliegenden Lewissäure hydrolysiert.

17. Verfahren zur Herstellung von 5-Cyano-5H-dibenz [b,f] azepin nach einem der Ansprüche 1-10, dadurch gekennzeichnet, dass man 5H-Dibenz [b,f] azepin mit einem Halogencyan in Gegenwart von stark polaren Substanzen umsetzt.

**Claims**

1. Process for producing 5H-dibenz [b,f] azepine-5-carboxamide, characterised in that 5H-dibenz [b,f] azepine is reacted with a halocyanogen in the presence of strongly polar substances to 5-cyano-5H-dibenz [b,f] azepine, and this is hydrolysed.

2. Process according to claim 1, characterised in that the halocyanogen used is cyanogen iodide, particularly cyanogen chloride or cyanogen bromide.

3. Process according to claim 1, characterised in that the strongly polar compounds used are aliphatic N-lower-alkyl- or N,N-di-lower-alkylamides of carboxylic acids of the formula R—COOH (I), in which lower alkyl can in each case be substituted by lower alkoxy, and lower alkyl and lower alkoxy each contain up to 7 carbon atoms, and R is lower alkyl having up to 5, especially up to 3, carbon atoms.

4. Process according to claim 1, characterised in that the strongly polar compounds used are N-lower-alkyl-phosphoric acid amides, in which lower alkyl has up to 4 carbon atoms.

5. Process according to claim 1, characterised in that the strongly polar substances used are optionally N-lower alkylated cyclic carboxylic acid imides having 5-7 ring members, in which lower alkyl has up to 4 carbon atoms.

6. Process according to claim 1, characterised in that the strongly polar substances used are mono-, di-, tri- or tetraalkylammonium compounds, wherein each of the alkyl groups has up to 18 carbon atoms, and can be optionally substituted by an aryl group which is unsubstituted or substituted by lower alkyl or lower alkoxy each having up to 7 carbon atoms, or by halogen, hydroxyl or nitro, or salts thereof, or poly-lower-alkylated urea, wherein lower alkyl has up to 3 carbon atoms, or sulfolane.

7. Process according to any one of claims 1-6, characterised in that the reaction is performed in admixture with a nonpolar solvent.

8. Process according to any one of claims 1-7, characterised in that the halocyanogen used is cyanogen chloride or cyanogen bromide, and the strongly polar substances used are N,N-di-lower-alkylamides of carboxylic acids of the formula (I), in which lower alkyl in each case has up to 3 carbon atoms, and R has up to 2 carbon atoms, also hexamethylphosphoric acid triamide, N-methylpyrrolidone-(2) or sulfolane, or, as quaternary ammonium compound, benzyltriethylammonium chloride or dibenzyldiethylammonium chloride, and optionally, as a nonpolar further solvent, 1,2-dichloroethane or 1,1,1-trichloroethane, or, as an aromatic solvent, benzene or toluene.

9. Process according to any one of claims 1-7, characterised in that the halocyanogen used is cyanogen chloride, and the strongly polar substances used are N,N-dimethylacetamide, N,N-diethylacetamide, also hexamethylphosphoric acid triamide or sulfolane, or, as quaternary ammonium compound, benzyltriethylammonium chloride, and optionally, as a nonpolar further solvent, 1,2-dichloroethane, 1,1,1-trichloroethane or toluene.

10. Process according to any one of claims 1-9, characterised in that the reaction with a halocyanogen is performed within a temperature range of 20-100 °C.

11. Process according to claim 1, characterised in that hydrolysis is performed with basic agents.

12. Process according to claim 11, characterised in that hydrolysis is performed by means of an oxide or hydroxide of alkaline-earth metals or alkali metals, optionally in the presence of a peroxide.

13. Process according to claim 12, characterised in that hydrolysis is performed by means of an alkali bicarbonate in admixture with hydrogen peroxide.

14. Process according to claim 1, characterised in that hydrolysis is performed by means of acid agents.

15. Process according to claim 1, characterised in that hydrolysis is performed by means of a Lewis acid optionally present as solution in a lower alkanecarboxylic acid.

16 Process according to claim 15, characterised in that hydrolysis is performed by means of a Lewis acid present as defined compound of the formula $BF_3 \cdot 2\ CH_3COOH$.

17. Process for producing 5-cyano-5H-dibenz [b,f] azepine according to any one of claims 1-10, characterised in that 5H-dibenz [b,f] azepine is reacted with a halocyanogen in the presence of strongly polar substances.

## Revendications

1. Procédé de préparation de 5H-dibenz [b,f] azépine-5-carboxamide, caractérisé en ce qu'on fait réagir la 5H-dibenz [b,f] azépine avec un cyanure d'halogène en présence de substances fortement polaires pour donner la 5-cyano-5H-dibenz [b,f] azépine et en ce qu'on hydrolyse celle-ci.

2. Procédé selon la revendication 1, caractérisé en ce qu'on utilise comme cyanure d'halogène le cyanure d'iode, en particulier le cyanure de chlore ou de brome.

3. Procédé selon la revendication 1, caractérisé en ce qu'on utilise comme composés fortement polaires des N-alcoyle inférieur- ou N,N-dialcoyle inférieur-amides aliphatiques d'acides carboxyliques de formule R—COOH (I), où alcoyle inférieur peut être à chaque fois substitué par un alcoxy inférieur, et alcoyle inférieur et alcoxy inférieur présentent respectivement jusqu'à 7 atomes de carbone et R représente un alcoyle inférieur ayant jusqu'à 5, en particulier jusqu'à 3, atomes de carbone.

4. Procédé selon la revendication 1, caractérisé en ce qu'on utilise comme composés fortement polaires des amides d'acides N-alcoyle inférieur-phosphoriques, où alcoyle inférieur a jusqu'à 4 atomes de carbone.

5. Procédé selon la revendication 1, caractérisé en ce qu'on utilise comme substances fortement polaires des imides d'acides carboxyliques cycliques avec substitution éventuelle de N-alcoyle inférieur et ayant de 5 à 7 chaînons, où alcoyle inférieur a jusqu'à 4 atomes de carbone.

6. Procédé selon la revendication 1, caractérisé en ce qu'on utilise comme substances fortement polaires des composés de mono-, di-, tri- ou tétra-alcoylammonium, où chacun des radicaux alcoyle a jusqu'à 18 atomes de carbone et peut être éventuellement substitué par un radical aryle éventuellement substitué par un alcoyle inférieur ou un alcoxy inférieur ayant respectivement jusqu'à 7 atomes de carbone, un halogène, un hydroxy ou un nitro, ou leurs sels, ou des urées à substitution poly-alcoyle inférieur, où alcoyle inférieur a jusqu'à 3 atomes de carbone, ou le sulfolane.

7. Procédé selon l'une des revendications 1-6, caractérisé en ce qu'on conduit la réaction en mélange avec un solvant non polaire.

8. Procédé selon l'une des revendications 1-7 caractérisé en ce qu'on utilise comme cyanure d'halogène du cyanure de chlore ou de brome et comme substances fortement polaires des N,N-dialcoyle inférieur-amides d'acides carboxyliques de formule (I), où alcoyle inférieur a respectivement jusqu'à 3 et R jusqu'à 2 atomes de carbone, ou encore le triamide de l'acide hexaméthylphosphorique, la N-méthylpyrrolidone-(2) ou le sulfolane, ou comme composé d'ammonium quaternaire le chlorure de benzyltriéthylammonium ou le chlorure de dibenzyl-diéthylammonium, et le cas échéant comme autres solvants non polaires le 1,2-dichloroéthane, le 1,1,1-trichloroéthane ou comme solvants aromatiques le benzène ou le toluène.

9. Procédé selon l'une des revendications 1-7, caractérisé en ce qu'on utilise comme cyanure d'halogène le cyanure de chlore et comme substances fortement polaires le N,N-diméthylacétamide, le N,N-di-éthylacétamide, ou encore le triamide de l'acide hexaméthylphosphorique ou le sulfolane, ou comme composé d'ammonium quaternaire le chlorure de benzyltriéthylammonium, et le cas échéant comme autres solvants non polaires le 1,2-dichloroéthane, le 1,1,1-trichloroéthane ou le toluène.

10. Procédé selon l'une des revendications 1-9, caractérisé en ce qu'on conduit la réaction avec un cyanure d'halogène dans un intervalle de températures de 20-100 °C.

11. Procédé selon la revendication 1, caractérisé en ce qu'on hydrolyse avec un agent basique.

12. Procédé selon la revendication 11, caractérisé en ce qu'on hydrolyse un moyen d'un oxyde ou hydroxyde de métaux alcalino-terreux ou alcalins, le cas échéant en présence d'un peroxyde.

13. Procédé selon la revendication 12, caractérisé en ce qu'on hydrolyse au moyen d'un bicarbonate alcalin mélangé à du peroxyde d'hydrogène.

14. Procédé selon la revendication 1, caractérisé en ce qu'on hydrolyse avec un agent acide.

15. Procédé selon la revendication 1, caractérisé en ce qu'on hydrolyse au moyen d'un acide de Lewis éventuellement présent sous forme de solution dans un acide alcane inférieur-carboxylique.

16. Procédé selon la revendication 15, caractérisé en ce qu'on hydrolyse au moyen de l'acide de Lewis présent sous la forme du composé défini de formule $BF_3 \cdot 2\ CH_3COOH$.

17. Procédé de préparation de 5-cyano-5H-dibenz [b,f]-azépine selon l'une des revendications 1-10, caractérisé en ce qu'on fait réagir de la 5H-dibenz [b,f] azépine avec un cyanure d'halogène en présence de substances fortement polaires.